# EUROPÄISCHE PATENTSCHRIFT

(11) **EP 2 862 562 B1**
(45) Veröffentlichungstag und Bekanntmachung des Hinweises auf die Patenterteilung: **28.12.2016**
(21) Anmeldenummer: 14157686.8
(22) Anmeldetag: 04.03.2014
(51) Int. Cl.: A61K 8/362, A61Q 19/02, A61K 8/97

(54) **Kosmetisches oder dermatologisches Mittel zur Aufhellung und zur Vermeidung von Auftreten der Hautflecken**
Cosmetic or dermatological composition for lightening and for preventing skin blemishes
Produit cosmétique ou dermatologique destiné à l'éclaircissement et à la prévention de l'apparition de taches sur la peau

(30) Priorität: 09.10.2013 DE 102013220352
(43) Veröffentlichungstag der Anmeldung: 22.04.2015
(73) Patentinhaber: Henkel AG & Co. KGaA, 40589 Düsseldorf (DE)
(72) Erfinder: Hartmann, Ira, 40589 Düsseldorf (DE); Waldmann-Laue, Marianne, 40789 Monheim (DE); Heinen, Soraya, 50858 Köln (DE)

(56) Entgegenhaltungen:
- EP-A2- 2 163 238
- WO-A1-03/039502
- WO-A1-2007/003289
- DE-A1- 10 238 449
- JP-A- 2001 172 157
- JP-A- 2003 081 850
- ROBINS R J ET AL: "High-performance liquid chromatographic methods for the analysis and purification of quassinoids from Quassia amara L", JOURNAL OF CHROMATOGRAPHY, ELSEVIER SCIENCE PUBLISHERS B.V, NL, Bd. 283, 1. Januar 1984 (1984-01-01), Seiten 436-440, XP026695100, ISSN: 0021-9673, DOI: 10.1016/S0021-9673(00)96287-3 [gefunden am 1984-01-01]

## Beschreibung

Die vorliegende Erfindung betrifft kosmetische oder dermatologische Mittel zur kosmetischen und topischen dermatologischen Hautaufhellung oder zur Verhinderung der Hautbräunung, insbesondere der durch UV-Strahlung hervorgerufenen Hautbräunung, sowie zur Aufhellung von Altersflecken oder Sommersprossen. Die vorliegende Erfindung betrifft weiterhin die Verwendung dieser Mittel und Verfahren unter Einsatz dieser Mittel.

Die Pigmentierung der Haut erfolgt durch Melanozyten, welche in der untersten Schicht der Epidermis neben den Basalzellen als je nach Hauttyp entweder vereinzelt oder gehäuft auftretende pigmentbildende Zellen vorzufinden sind. Melanozyten enthalten Melanosomen, in denen Melanin gebildet wird. Durch verschiedene chemische und/oder physikalische Einflüsse, insbesondere durch UV-Strahlung, wird verstärkt Melanin gebildet. Dieses wird über die Keratinozyten in die Corneozyten (Hornschicht) transportiert und führt zu einer bräunlichen bis braun-schwarzen Hautfarbe. Melanin wird als Endstufe eines oxidativen Prozesses gebildet, in welchem Tyrosin unter Mitwirkung der Enzyms Tyrosinase über mehrere Zwischenstufen zu den braun bis braunschwarzen Eumelaninen (DHICA- und DHI-Melanin) bzw. unter Beteiligung von schwefelhaltigen Verbindungen zum rötlichen Phäomelanin umgewandelt wird. Die Melaninbildung - und damit Hautfarbe - unterliegt äußeren Einflüssen und kann neben erwünschten Effekten ("gesunde Bräune") auch zu unerwünschten Erscheinungen führen. So kann z.B. UV-Strahlung zu Sommersprossen führen. Fehlpigmentierungen können auch aufgrund genetischer Disposition, Wundheilung bzw. -vernarbung oder Hautalterung ("Altersflecken") auftreten. Da der Prozess der Desquamation (oberflächliche Loslösung von Zellen oder Zellgruppen aus ihrem epithelialen Verband) einen beständigen Verlust an Melanin beinhaltet, kann eine Aufhellung der Haut durch Inhibierung der Neusynthese von Melanin erreicht werden.

Altersflecken stellen ein Zeichen der Hautalterung dar, das einer speziellen Behandlung bedarf. Bei Altersflecken handelt es sich nicht um eine intensivere Pigmentierung der Haut, sondern konkret um Anhäufungen des bräunlich-wachsartigen Pigments Lipofuscin (auch Alters- oder Abnutzungspigment), das als Endprodukt aus der Oxidation von ungesättigten Fettsäuren der Zellmembranen entsteht. Die Lysosomen sind nicht mehr imstande, den Stoff vollständig abzubauen. So bleibt er als Fleck zurück.

Wirksame kosmetische Mittel zur Hautaufhellung sind bekannt. Diese enthalten als Wirkstoff jedoch zumeist Hydrochinon oder Kojisäure (zur Inhibierung der Tyrosinase). Beide Substanzen sind mutagen und sollten daher nicht über längere Zeiträume verwendet werden. Hautkosmetische Mittel zur Behandlung von Altersflecken werden beispielsweise in der europäischen Patentanmeldung EP 2163238 A2 beschrieben.

Es bestand nach wie vor die Aufgabe, lagerstabile hautaufhellende kosmetische oder dermatologische Mittel zur Verfügung zu stellen, die hochwirksam, auch unter UV-Bestrahlung (Sonnenlicht) anwendbar und möglichst auf Naturstoffen basiert sind. Darüber hinaus sollten weitere positive Effekte auf älterer Haut ermöglicht werden, z.B Hautstraffung und/oder Reduktion der Hautfalten.

Es hat sich überraschenderweise gezeigt, dass die Kombination von mindestens einem Extrakt aus Quassia Amara, mit mindestens einer Dicarbonsäure, ausgewählt aus 9-Octadecen-1,18-dicarbon-säure, 8-Hexadecen-1,16-dicarbonsäure, 7-Tetradecen-1,14-dicarbonsäure, 6-Dodecen-1,12-dicarbonsäure, 5-Decen-1,10-dicarbonsäure, Sebacinsäure und Azelainsäure sowie Mischungen dieser Dicarbonsäuren, zu einer besonders guten Aufhellung der Hautfarbe führt, sowohl bei großflächiger Anwendung auf der Haut als auch bei lokaler Anwendung auf Pigmentstörungen wie Altersflecken.

Ein erster Gegenstand der vorliegenden Erfindung sind daher kosmetische oder dermatologische Mittel, enthaltend eine Kombination aus mindestens einem der unter a) und mindestens einem der unter b) aufgeführten Wirkstoffe:
a) Extrakt aus Quassia Amara, welcher durch Extraktion von Quassia amara Holz mit Wasser erhältlich ist;
b) gesättigte oder ungesättigte, lineare (C9- bis C18)-Dicarbonsäure, insbesondere 8-Hexadecen-1,16-dicarbonsäure und/oder 7-Tetradecen-1,14-dicarbonsäure und/oder 9-Octadecen-1,18-dicarbonsäure und/oder 6-Dodecen-1,12-dicarbonsäure und/oder 5-Decen-1,10-dicarbonsäure und/oder Decandisäure (Sebacinsäure) und/oder Nonandisäure (Azelainsäure), ganz besonders bevorzugt 8-Hexadecen-1,16-dicarbonsäure.

Die erfindungsgemäßen Mittel enthalten als erste obligatorische Komponente, bezogen auf das Gesamtgewicht der erfindungsgemäßen Mittel, insgesamt 0,001 bis 2,0 Gew.-%, vorzugsweise 0,01 bis 0,5 Gew.-% und insbesondere 0,05 bis 0,2 Gew.-% mindestens eines Extraktes aus Quassia Amara, welcher durch Extraktion von Quassia amara Holz mit Wasser erhältlich ist.

Der Quassia amara, Brasilianischer Quassiabaum, Quassiabaum, Quassiaholzbaum, Bitterquassia oder wie einige andere Arten auch Bitterholz genannt, ist eine Pflanzenart in der Familie der Bittereschengewächse (Simaroubaceae). Sie ist in der Neotropis weit verbreitet. Die erfindungsgemäß eingesetzten Extrakte werden aus dem Holz gewonnen.

Extrakt aus Quassia amara Holz können nach bekannten Herstellungsverfahren in variabler Zusammensetzung mit Lösungsmitteln wie z. B. Wasser, Methanol, Ethanol, Aceton, etc., und deren Gemische bei Temperaturen von Raumtemperatur bis 100 °C unter gelinder bis heftiger Durchmischung innerhalb von 10 Min. bis 24 Std. unter Normaldruck bis zu 200 bar erhalten werden. Zur Anreicherung wirksamkeitsbestimmender Komponenten können weitere Konzentrierungsschritte durchgeführt werden wie z. B. flüssig-flüssig-Verteilung mit z. B. 1-Butanol/Wasser oder Ethylacetat/Wasser, Adsorption-Desorption an Ionenaustauscher, LH20, HP20 und andere Harze oder chromatographische Abtrennungen über RP18, Kieselgel, etc.. Falls die Weiterverarbeitung zu Trockenextrakten erwünscht ist, erfolgt diese nach an sich bekannten Verfahren durch Abziehen des Lösungsmittels bei erhöhter Temperatur und / oder reduziertem Druck. Die Angaben zum Gewichtsanteil von Quassia Amara Holz am Gesamtgewicht der erfindungsgemäßen Mittel beziehen sich auf die Trockenmasse des Extraktes.

Erfindungsgemäß ist ein Extrakt, der durch Extraktion von Quassia amara Holz mit Wasser erhältlich ist.

Das Handelsprodukt des Extraktes aus Quassia Amara Holz kann außer Extraktionsmittel noch zusätzliche Lösungsmittel zur Verbesserung der Haltbarkeit des Produkts enthalten. Propylenglycol, 1,3 - Butylenglycol und Pentylenglycol sind beispielweise solche antimikrobiellen Lösungsmittel.

Bevorzugte erfindungsgemäße kosmetische oder dermatologische Mittel enthalten, bezogen auf ihr Gesamtgewicht, 0,001 bis 2,0 Gew.-%, vorzugsweise 0,01 bis 0,5 Gew.-% und insbesondere 0,05 bis 0,2 Gew.-% Pentylenglycol.

Weitere bevorzugte erfindungsgemäße kosmetische oder dermatologische Mittel enthalten, bezogen auf ihr Gesamtgewicht, 0,003 bis 6,0 Gew.-%, vorzugsweise 0,03 bis 1,5 Gew.-% und insbesondere 0,15 bis 0,6 Gew.-% 1,3 - Butylenglycol.

Die erfindungsgemäßen Mittel mit Extrakt aus Quassia amara Holz zeigen im Vergleich zur Mittelvariante, die kein Quassia amara Extrakt enthält, viel bessere Aufhellungseffekt gegen Hautflecken und schützten die Haut wirksamer gegen das Auftreten neuer Hautflecken.

Als zweite obligatorische Komponente enthalten die erfindungsgemäßen Mittel mindestens eine gesättigte oder ungesättigte, lineare (C9- bis C18)-Dicarbonsäure, insbesondere 8-Hexadecen-1,16-dicarbonsäure und/oder 7-Tetradecen-1,14-dicarbonsäure und/oder 9-Octadecen-1,18-dicarbonsäure und/oder 6-Dodecen-1,12-dicarbonsäure und/oder 5-Decen-1,10-dicarbonsäure und/oder Decandisäure (Sebacinsäure) und/oder Nonandisäure (Azelainsäure), ganz besonders bevorzugt 8-Hexadecen-1,16-dicarbonsäure.

Die erfindungsgemäßen Mittel enthalten, bezogen auf ihr Gesamtgewicht, die gesättigte oder ungesättigte, lineare (C9- bis C18)-Dicarbonsäure, insbesondere die 8-Hexadecen-1,16-dicarbonsäure, in einer Gesamtmenge von 0,01 - 10 Gew.-%, vorzugsweise 0,05 bis 1 Gew.-% und insbesondere 0,1 bis 0,5 Gew.-%.

8-Hexadecen-1,16-dicarbonsäure (Dioic acid, CAS-Nummer 20701-68-2), die auch als 9-Octadecen-1,18-disäure bezeichnet wird, ist ein Stoffwechselprodukt von Hefezellen aus selektierten Mutanten-Stämmen des Candida Stammes, wobei als Ausgangssubstanz eine Fettsäure rein pflanzlichen Ursprungs dient, die in die Hydroxyfettsäure umgesetzt wird, welche anschließend über die Stufe des Fettsäurealdehyd zur Dicarboxysäure oxidiert wird. 8-Hexadecen-1,16-dicarbonsäure ist durch folgende Struktur gekennzeichnet: 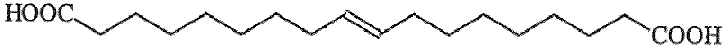 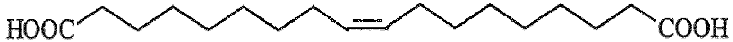 8-Hexadecen-1,16-dicarbonsäure ist unter dem Handelsnamen ODA White (INCI-Bezeichnung OCTADECENEDIOIC ACID, BHT) von Croda (Sederma) erhältlich. Das Handelsprodukt besitzt eine Reinheit von 100%, wobei die 8- Hexadecen-1,16-dicarbonsäure darin als Gemisch des cis- und trans- Isomeren vorliegt und das cis-Isomere mengenmäßig überwiegt.

Vorzugsweise werden der mindestens eine Extrakt aus Quassia Amara Holz und die mindestens eine gesättigte oder ungesättigte, lineare (C9- bis C18)-Dicarbonsäure in bestimmten Mengenverhältnissen zueinander eingesetzt. Hier sind erfindungsgemäße kosmetische oder dermatologische Mittel bevorzugt, bei denen das Gewichtsverhältnis des Gesamtgehalts an Extrakt aus Quassia Amara Holz zur Gesamtmenge der gesättigten oder ungesättigten, linearen (C9- bis C18)-Dicarbonsäure 1 : 10 bis 5: 1, vorzugsweise 1 : 5 bis 2 : 1 und insbesondere 1 : 3 bis 1 : 1, beträgt. Bei der Bestimmung des Gewichtsverhältnisses kommt nur Trockenmasse des Extraktes aus Quassia Amara Holz in Betracht.

In einer bevorzugten Ausführungsform enthalten die erfindungsgemäßen Mittel, zur Unterstützung der aufhellenden Wirkung der Kombination von mindestens einem Extrakt aus Quassia Amara Holz mit mindestens einer gesättigten oder ungesättigten, linearen (C9- bis C18)-Dicarbonsäure, bezogen auf das Gesamtgewicht der erfindungsgemäßen Mittel, zusätzlich insgesamt 0,01 bis 5 Gew.-%, vorzugsweise 0,1 bis 1 Gew.-%, an mindestens einer weiteren hautaufhellenden Substanz, die ausgewählt ist aus der Gruppe, bestehend aus Ascorbinsäure, Ascorbinsäuresalzen, Ascorbinsäureglycosiden, Ascorbinsäureglycosidsalzen, Ascorbinsäureestern von organischen Säuren, den Salzen der Ascorbinsäureester von organischen Säuren, Ascorbinsäureestern von anorganischen Säuren, den Salzen der Ascorbinsäureester von anorganischen Säuren, sowie Mischungen der vorgenannten Substanzen, insbesondere Ascorbinsäure.

Unter Ascorbinsäuresalzen sind die Alkali- und Erdalkalisalze bevorzugt. Besonders bevorzugte Salze der Ascorbinsäure, die erfindungsgemäß eingesetzt werden können, sind Natriumhydroascorbat, Natriumascorbat, Kaliumhydroascorbat, Kaliumascorbat, Ammoniumascorbat, Ammoniumhydroascorbat, Calciumascorbat, Calciumhydroascorbat, Magnesiumascorbat und Magnesiumhydroascorbat.

Mit besonderem Vorzug können auch die Ester der Ascorbinsäure eingesetzt werden. Unter den Estern der Ascorbinsäure sind insbesondere Ascorbylmethanoat, Ascorbylethanoat, Ascorbyl-n-propanoat, Ascorbyl-iso-propanoat, Ascorbyl-n-butanoat, usw. bevorzugt. Mit besonderem Vorzug enthalten die erfindungsgemäßen Mittel Ester von Ascorbinsäure mit Fettsäuren, insbesondere Ascorbyllinoleat, Ascorbyloleat, Ascorbyloleinat, Ascorbylpalmitat, Ascorbylstearat, Ascorbyloleat (6-Palmitoyl-L-ascorbinsäure) und Ascorbyltetraisopalmitat.

Auch "anorganische" Säuren können mit Ascorbinsäure verestert werden. Unter den "anorganischen" Estern der Ascorbinsäure ist insbesondere das Ascorbylphosphat bevorzugt.

Auch Ascorbinsäurederivate mit glycosidisch gebundenen Zuckern sind erfindungsgemäß mit besonderem Vorzug einsetzbar. Bewährt hat sich hier insbesondere das Ascorbylglucosid.

Bevorzugt sind erfindungsgemäße kosmetische oder dermatologische Mittel, die Ascorbylphosphat und/oder Ascorbylglucosid und/oder Ascorbyltetraisopalmitat enthalten.

Abgesehen von der Ascorbinsäure und/oder Ascorbinsäuresalzen und/oder Ascorbinsäurederivaten können die erfindungsgemäßen Mittel in einer weiteren bevorzugten Ausführungsform weiterhin wenigstens einen pflegenden Wirkstoff, ausgewählt aus der Gruppe der Vitamine, Provitamine oder Vitaminvorstufen, enthalten. Diese sind in den erfindungsgemäßen Mitteln in einer Menge von 0,1 - 10 Gew.-%, vorzugsweise 0,2 - 5 Gew.-% und insbesondere 0,5 - 1 Gew.-%, jeweils bezogen auf das Gesamtgewicht der Mittel, enthalten. Dabei sind erfindungsgemäß solche Vitamine, Pro-Vitamine und Vitaminvorstufen bevorzugt, die üblicherweise den Gruppen A, B, F und H zugeordnet werden.

Zur Gruppe der als Vitamin A bezeichneten Substanzen gehören das Retinol (Vitamin A1) sowie das 3,4-Didehydroretinol (Vitamin A2). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht.

Zur Vitamin B-Gruppe oder Vitamin B-Komplex gehören u. a.
- Vitamin B1 (Thiamin)
- Vitamin B2 (Riboflavin)
- Vitamin B3. Unter dieser Bezeichnung werden häufig die Verbindungen Nicotinsäure und Nicotinsäureamid (Niacinamid) geführt, unter denen insbesondere das Nicotinsäureamid erfindungsgemäß bevorzugt ist.
- Vitamin B5 (Pantothensäure und Panthenol). Im Rahmen dieser Gruppe wird bevorzugt das Panthenol eingesetzt. Erfindungsgemäß einsetzbare Derivate des Panthenols sind insbesondere die Ester und Ether des Panthenols, kationisch derivatisierte Panthenole sowie Pantolacton.
- Vitamin B6 (Pyridoxin sowie Pyridoxamin und Pyridoxal).

Unter dem Begriff "Vitamin F" werden üblicherweise essentielle Fettsäuren, insbesondere Linolsäure, Linolensäure und Arachidonsäure, verstanden.

Als Vitamin H wird die Verbindung (3aS, 4S, 6aR)-2-Oxohexahydrothienol[3,4-d]-imidazol-4-valeriansäure bezeichnet, für die sich aber zwischenzeitlich der Trivialname Biotin durchgesetzt hat.

Es ist auch vorteilhaft im Sinne der vorliegenden Erfindung, kosmetische oder dermatologische Mittel zu erstellen, deren hauptsächlicher Zweck nicht der Schutz vor Sonnenlicht ist, die aber dennoch einen Gehalt an weiteren UV-Schutzsubstanzen enthalten.

Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. Die UVA-, UVB- und UVC-Filter können sowohl einzeln als auch in Mischungen eingesetzt werden. UV-Filter mit einem Absorptionsmaximum im UVB-Bereich, insbesondere im Bereich von etwa 280 bis etwa 300 nm, sind besonders bevorzugt.

Der Einsatz von mindestens einer anorganischen und/oder mindestens einer organischen UV-Filtersubstanz ist erfindungsgemäß bevorzugt.

Die erfindungsgemäß bevorzugten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäßen kosmetischen Zubereitungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethyl-hexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydroxybenzoyl)-benzoesäurehexylester (auch: Aminobenzophenon, unter der Bezeichnung Uvinul A Plus bei der Firma BASF erhältlich), 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/Dimethylsiloxan-Copolymer mit der INCI-Bezeichnung Dimethicodiethylbenzal malonate (CAS-Nr. 207574-74-1, Parsol^{®} SLX), Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxyphenol Methoxyphenyl Triazin, unter dem Namen Tinosorb S bei CIBA erhältlich), Dioctylbutylamidotriazon (INCI: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvinul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma), die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((tri-methylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2-propyloxy)-2-hydroxy-propyloxy)-2-hydroxy]-phenyl}-6-[4-(ethylcarboxyl)-phenylamino]-1,3,5-triazin, 2,4-Bis-{[4-(2-ethylhexyloxy)-2-hydroxy]-phenyl}-6-(1-methyl-pyrrol-2-yl)-1,3,5-triazin, 2,4-Bis-{[4-tris(trimethylsiloxy-silylpropyloxy)-2-hydroxy]-phenyl}-6-(4-methoxyphenyl)-1,3,5-triazin, 2,4-Bis-{[4-(2-methylpropenyloxy)-2-hydroxy]-phenyl}-6-(4-methoxyphenyl)-1,3,5-triazin, 2,4-Bis-{[4-(1', 1', 1',3',5',5',5'-Heptamethylsiloxy-2-methyl-propyloxy)-2-hydroxy]-phenyl}-6-(4-methoxyphenyl)-1,3,5-triazin sowie Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, insbesondere die Sulfonsäure selbst mit der INCI-Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalydene Dicampher Sulfonic Acid (CAS.-Nr.: 90457-82-2, als Mexoryl SX von der Firma Chimex erhältlich).

Auch anorganische Pigmente können erfindungsgemäß als UV-Filter eingesetzt werden. Bevorzugte anorganische Pigmente sind Metalloxide und/oder andere in Wasser schwerlösliche oder unlösliche Metallverbindungen, beispielsweise Carbonate, Sulfate usw. Bevorzugt sind insbesondere Oxide des Titans (TiO2), Zinks (ZnO), Eisens (z. B. Fe 2O3), Zirkoniums (ZrO2), Siliciums (SiO 2), Mangans (z. B. MnO), Aluminiums (Al2O3), Cers (z. B. Ce2O3), Mischoxide der entsprechenden Metalle sowie Abmischungen aus solchen Oxiden sowie das Sulfat des Bariums (BaSO4).

Die anorganischen Pigmente können einen mittleren Teilchendurchmesser von maximal 200 µm haben und aus den Oxiden von Silicium, Titan, Eisen, Zink, Zirkonium, Magnesium, Cer und Bismut, aus Bornitrid, Glimmer, Flussspat und wasserunlöslichen Perlglanzpigmenten, die mit mindestens einer anorganischen und/oder organischen Verbindung beschichtet sein können, ausgewählt sein. Bevorzugte anorganische Pigmente weisen einen Durchmesser von 1 bis 100 µm auf, besonders bevorzugt 1 bis 50 µm, und außerordentlich bevorzugt 1 bis 10 µm.

Die Pigmente können sowohl farbig als auch farblos sein. Die bevorzugten Pigmente sind ausgewählt aus den Oxiden von Silicium, Titan, Zink und Eisen, sowie aus Bismutoxychlorid. Weitere erfindungsgemäß bevorzugte Pigmente sind Glimmer und Perlglanzpigmente.

Die genannten anorganischen Pigmente können beschichtet sein. Die Beschichtung kann mit Hilfe von anorganischen und/oder organischen Verbindungen erfolgen. Erfindungsgemäß bevorzugt sind anorganische Pigmente, die eine anorganische Beschichtung aufweisen.

Bevorzugte Pigmente dieser Art sind ausgewählt aus Siliciumdioxid-Partikeln, die mit Titandioxid und/oder Eisenoxiden beschichtet sind. Ein besonders bevorzugtes Pigment dieser Art ist das Handelsprodukt Ronasphere®LDP der Firma Merck KGaA. Bei diesem Produkt handelt es sich um sphärische Siliciumdioxid-Partikel, die mit Titandioxid und Eisenoxid beschichtet sind. Ronasphere®LDP weist einen Brechungsindex auf, der dem Brechungsindex der Haut ähnelt.

Weiterhin geeignet sind anorganisch beschichtete Glimmerpigmente, die keinen Perlglanz aufweisen.

Weitere bevorzugte anorganisch beschichtete anorganische Pigmente sind Glimmerpigmente, die mit Titandioxid in verschiedenen Schichtdicken beschichtet sind, beispielsweise das Produkt RonaFlair® Extender W von Merk KGaA. Das genannte Produkt wies durchschnittliche Teilchendurchmesser von 3 - 8 µm auf. Ebenfalls bevorzugt sind Glimmerpartikel mit einer Beschichtung aus Metalloxidmischung. Weitere erfindungsgemäß geeignete Pigmente sind anorganisch beschichtete anorganische Pigmente, deren Beschichtung einen Anteil von 0,1 - 5 Gew.-% Zinnoxid aufweist.

Ebenfalls erfindungsgemäß geeignet, wenngleich weniger bevorzugt, sind anorganische Pigmente, die mit organischen Substanzen beschichtet sind. Beispiele hierfür sind mit Aluminiumstearat beschichtete Titandioxid-Pigmente, mit Dimethylpolysiloxan (Dimethicone) beschichtetes Zinkoxid, mit Dimethicone beschichtetes Bornitrid, mit einem Gemisch aus Dimethylpolysiloxan und Silicagel (Simethicone) und Aluminiumoxidhydrat (Alumina) beschichtetes Titandioxid, mit Octylsilanol beschichtetes Titandioxid oder sphärische Polyalkylsesquisiloxan-Partikel.

In einer bevorzugten Ausführungsform enthalten die erfindungsgemäßen Mittel mindestens ein Metalloxid, vorzugsweise ein Metalloxid als hautaufhellenden Wirkstoff. Hier sind erfindungsgemäße kosmetische oder dermatologische Mittel bevorzugt, die bezogen auf ihr Gesamtgewicht, insgesamt 0,01 bis 5 Gew.-%, vorzugsweise 0,1 bis 3 Gew.-% Metalloxid, bevorzugt Metalloxid als hautaufhellenden Wirkstoff enthalten. Erfindungsgemäß vorzugsweise einsetzbar ist Titandioxid.

Die erfindungsgemäßen Mittel enthalten die jeweiligen Wirkstoffe in einem kosmetisch verträglichen Träger. Dieser kosmetische Träger ist bevorzugt wässrig, alkoholisch, wässrigalkoholisch oder wässrig-glycolisch. Solche Träger sind beispielsweise Cremes, Emulsionen, Gele oder auch tensidhaltige schäumende Lösungen, wie beispielsweise Duschgele oder andere Zubereitungen, die für die Anwendung auf der Haut geeignet sind. Ein wässriger Träger enthält im Sinne der Erfindung bevorzugt mindestens 40 Gew.-%, besonders bevorzugt mindestens 50 Gew.-% Wasser. Erfindungsgemäß bevorzugte kosmetische Mittel sind dadurch gekennzeichnet, daß sie, bezogen auf das Gesamtgewicht des anwendungsbereiten Mittels 10 bis 80 Gew.-%, vorzugsweise 20 bis 75 Gew.-%, weiter bevorzugt 30 bis 70 Gew.-%, noch weiter bevorzugt 35 bis 60 Gew.-% und insbesondere 40 bis 60 Gew.-% Wasser enthalten.

Unter wässrig-alkoholischen Trägern sind im Sinne der vorliegenden Erfindung wasserhaltige Zusammensetzungen, enthaltend 3 bis 70 Gew.-%, bevorzugt 10 - 60 Gew.-%, besonders bevorzugt 15 - 40 Gew.-%, eines einwertigen C2-C4-Alkohols, insbesondere Ethanol, zu verstehen.

Unter wässrig-glycolischen Trägern sind im Sinne der vorliegenden Erfindung wasserhaltige Zusammensetzungen, enthaltend 3 bis 70 Gew.-%, bevorzugt 10 - 60 Gew.-%, besonders bevorzugt 15 - 40 Gew.-%, mindestens eines zwei- oder mehrwertigen C2-C9-Alkohols oder mindestens eines wasserlöslichen Polyethylenglycols mit 3 - 20 Ethylenoxid-Einheiten, insbesondere ausgewählt aus 1,2-Propylenglycol, 2-Methyl-1,3-propandiol, Glycerin, 1,2-Butylenglycol, 1,3-Butylenglycol, 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, PEG-3, PEG-4, PEG-6, PEG-7, PEG-8, PEG-9, PEG-10, PEG-12, PEG-14, PEG-16, PEG-18 und PEG-20 sowie Mischungen hiervon, zu verstehen.

Die erfindungsgemäßen Mittel können zusätzlich weitere organische Lösemittel, wie beispielsweise Methoxybutanol, Ethyldiglycol, 1,2-Propylenglycol, 2-Methyl-1,3-propandiol, n-Propanol, n-Butanol, n-Butylenglycol, Glycerin, Diethylenglycolmonoethylether und Diethylenglycolmono-n-butylether, enthalten. Bevorzugt sind dabei alle wasserlöslichen organischen Lösemittel.

Die erfindungsgemäßen Mittel können in allen für die topische Applikation auf die Haut geeigneten Darreichungsformen konfektioniert sein. Bevorzugte Darreichungsformen sind eine Lösung, eine Emulsion vom Typ Wasser-in-Öl (W/O), eine Emulsion vom Typ Öl-in-Wasser (O/W), eine multiple Emulsion, beispielsweise vom Typ Wasser-in-Öl-in-Wasser (W/O/W) oder Öl-in-Wasser-in-Öl (O/W/O), eine Hydrodispersion, eine Lipodispersion, ein Gel, ein Hydrodispersionsgel, ein Lipodispersionsgel, ein fester Stift, ein Gelpflaster (Gelpatch), ein Pflaster, ein Kataplasma, Wirkstoff-haltige Liposomen oder ein transdermales therapeutisches System. Niedrig-viskose oder mittelviskose Darreichungsformen können auch in einem treibgasfreien Pump- oder Sprühspender oder zusammen mit einem Treibmittel in einem Aerosolbehälter konfektioniert sein.

Die anwendungsbereiten erfindungsgemäßen Mittel können weitere Wirk-, Hilfs- und Zusatzstoffe enthalten.

Beispielsweise hat es sich für die Applikation und den Verbleib des Mittels auf der Haut als vorteilhaft erwiesen, wenn die Mittel mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen. Geeignete Verdickungsmittel sind
- anionische, synthetische Polymere;
- kationische, synthetische Polymere;
- natürlich vorkommende Verdickungsmittel, wie Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Vorzugsweise werden die Mittel als fließfähige Zubereitungen bereitgestellt. "Fließfähig" im Sinne der vorliegenden Anmeldung sind dabei Zusammensetzungen, die gießbar sind und Viskositäten von 10 mPa·s bis hin zu 250.000 mPa·s (20 °C) aufweisen können. Die Viskosität kann mit üblichen Standardmethoden (beispielsweise Brookfield-Viskosimeter LVT-II bei 20 U/min und 20°C, Spindel 3) gemessen werden und liegt vorzugsweise im Bereich von 50 bis 50000 mPa·s. Bevorzugte Mittel haben Viskositäten von 1000 bis 30000 mPas, wobei Werte von 5000 bis 20000 mPas besonders bevorzugt sind.

Erfindungsgemäße Emulsionen im Sinne der vorliegenden Erfindung, z. B. in Form einer Creme, einer Lotion oder einer kosmetischen Milch, sind vorteilhaft und enthalten z. B. Fette, Öle, Wachse und/oder andere Fettkörper, sowie Wasser und einen oder mehrere Emulgatoren, wie sie üblicherweise für einen solchen Typ der Formulierung verwendet werden.

In einer weiteren bevorzugten Ausführungsform können die erfindungsgemäßen Mittel Emulgatoren enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C12-C22-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C8-C22-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phosphatidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls® PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 30 Gew.-%, insbesondere 3 - 15 Gew.-%, bezogen auf das gesamte Mittel.

Bevorzugt können die erfindungsgemäßen Mittel mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Die erfindungsgemäßen Mittel eignen sich zur Aufhellung der Haut und können dementsprechend eingesetzt werden. Dabei kann die Haut lokal begrenzt (z.B. auf Sommersprossen, Altersflecken oder Fehlpigmentierungen) oder großflächig (zur Hautaufhellung) behandelt werden.

Weitere Gegenstände der vorliegenden Erfindung sind daher die nicht-therapeutische, kosmetische Verwendung von erfindungsgemäßen kosmetischen oder dermatologischen Mitteln zur Aufhellung der Hautfarbe, zur Reduzierung der Pigmentierung der Haut, zum Ausgleich des Erscheinungsbildes einer ungleichmäßigen Hautpigmentierung und/oder zur Aufhellung von Altersflecken oder Sommersprossen der Haut.

Ein weiterer Gegenstand der vorliegenden Erfindung ist die nicht-therapeutische, kosmetische Verwendung von erfindungsgemäßen kosmetischen und dermatologischen Mittel zur Behandlung postinflammatorischer Hyperpigmentierung.

Bezüglich bevorzugter Ausführungsformen der erfindungsgemäßen Verwendungen gilt mutatis mutandis das zu den erfindungsgemäßen Mittel Gesagte.

Ein weiterer Gegenstand der vorliegenden Erfindung ist ein nicht-therapeutisches, kosmetisches Verfahren zur Aufhellung der Hautfarbe, zur Reduzierung der Pigmentierung der Haut, zum Ausgleich des Erscheinungsbildes einer ungleichmäßigen Hautpigmentierung und/oder zur Aufhellung von Altersflecken oder Sommersprossen der Haut, dadurch gekennzeichnet, dass ein kosmetisches Mittel, enthaltend eine Kombination aus mindestens einem der unter a) und mindestens einem der unter b) aufgeführten Wirkstoffe:
a) Extrakt aus Quassia Amara, welcher durch Extraktion von Quassia amara Holz mit Wasser erhältlich ist;
b) gesättigte oder ungesättigte, lineare (C9- bis C18)-Dicarbonsäure, insbesondere 8-Hexadecen-1,16-dicarbonsäure und/oder 7-Tetradecen-1,14-dicarbonsäure und/oder 9-Octadecen-1,18-dicarbonsäure und/oder 6-Dodecen-1,12-dicarbonsäure und/oder 5-Decen-1,10-dicarbonsäure und/oder Decandisäure (Sebacinsäure) und/oder Nonandisäure (Azelainsäure), ganz besonders bevorzugt 8-Hexadecen-1,16-dicarbonsäure,
auf die Haut aufgetragen wird.

Bezüglich bevorzugter Ausführungsformen der erfindungsgemäßen Verfahren gilt mutatis mutandis das zu den erfindungsgemäßen Mittel Gesagte.

Die folgenden Beispiele erläutern die Erfindung, ohne sie jedoch darauf einzuschränken:

### Beispiele:

Es wurden die folgenden kosmetischen oder dermatologischen Mittel hergestellt, wobei alle Zahlenwerte in den nachfolgenden Beispielen - sofern nicht anders angegeben - der Menge des jeweiligen Wirkstoffs in Gewichtsprozent bezogen auf das Gesamtgewicht des Mittels entsprechen:

| | **Formel 1** | **Formel 2** | **Formel 3** | **Formel 4** | **Formel 5** |
|---|---|---|---|---|---|
| **Extrakt Quassia Amara** | 0,001 bis 2 | 0,001 bis 2 | 0,01 bis 0,5 | 0,01 bis 0,5 | 0,05 bis 0,2 |
| **Gesättigte oder ungesättigte, lineare (C9-C18)-Dicarbonsäure** | 0,01 bis 10 | 0,05 bis 1 | 0,1 bis 0,8 | 0,1 bis 0,5 | 0,1 bis 0,5 |
| **Wasser und weitere Inhaltsstoffe** | add 100 | add 100 | add 100 | add 100 | add 100 |

| | **Formel 6** | **Formel 7** | **Formel 8** | **Formel 9** | **Formel 10** |
|---|---|---|---|---|---|
| **Extrakt Quassia Amara** | 0,001 bis 2 | 0,001 bis 2 | 0,01 bis 0,5 | 0,01 bis 0,5 | 0,05 bis 0,2 |
| **8-Hexadecen-1,16-dicarbonsäure** | 0,01 bis 6 | 0,02 bis 1 | 0,05 bis 0,8 | 0,05 bis 0,5 | 0,1 bis 0,4 |
| **Wasser und weitere Inhaltsstoffe** | add 100 | add 100 | add 100 | add 100 | add 100 |

| | **Formel 11** | **Formel 12** | **Formel 13** | **Formel 14** | **Formel 15** |
|---|---|---|---|---|---|
| **Extrakt Quassia Amara** | 0,001 bis 2 | 0,001 bis 2 | 0,01 bis 0,5 | 0,01 bis 0,5 | 0,05 bis 0,2 |
| **8-Hexadecen-1,16-dicarbonsäure** | 0,01 bis 6 | 0,02 bis 1 | 0,05 bis 0,8 | 0,05 bis 0,5 | 0,1 bis 0,4 |
| **Ascorbinsäure und/oder Ascorbinsäuresalze und/oder Ascorbinsäurederivate** | 0,01 bis 15 | 0,05 bis 5 | 0,1 bis 3 | 0,2 bis 2 | 0,3 bis 1 |
| **Wasser und weitere Inhaltsstoffe** | add 100 | add 100 | add 100 | add 100 | add 100 |

| | **Formel 16** | **Formel 17** | **Formel 18** | **Formel 19** | **Formel 20** |
|---|---|---|---|---|---|
| **Extrakt Quassia Amara** | 0,001 bis 2 | 0,001 bis 2 | 0,01 bis 0,5 | 0,01 bis 0,5 | 0,05 bis 0,2 |
| **Gesättigte oder ungesättigte, lineare (C9-C18)-Dicarbonsäure** | 0,01 bis 6 | 0,02 bis 1 | 0,05 bis 0,8 | 0,05 bis 0,5 | 0,1 bis 0,4 |
| **Magnesium Ascorbyl Phosphat** | 0,01 bis 7 | 0,03 bis 3 | 0,05 bis 1,5 | 0,1 bis 1 | 0,2 bis 0,6 |
| **Ascorbyl Tetraisopalmitat** | 0,01 bis 8 | 0,03 bis 4 | 0,05 bis 2 | 0,1 bis 1 | 0,2 bis 0,7 |
| **Wasser und weitere Inhaltsstoffe** | add 100 | add 100 | add 100 | add 100 | add 100 |

| | **Formel 21** | **Formel 22** | **Formel 23** | **Formel 24** | **Formel 25** |
|---|---|---|---|---|---|
| **Extrakt Quassia Amara** | 0,001 bis 2 | 0,001 bis 2 | 0,01 bis 0,5 | 0,01 bis 0,5 | 0,05 bis 0,2 |
| **8-Hexadecen-1,16-dicarbonsäure** | 0,01 bis 6 | 0,02 bis 1 | 0,05 bis 0,8 | 0,05 bis 0,5 | 0,1 bis 0,4 |
| **Magnesium Ascorbyl Phosphat** | 0,01 bis 7 | 0,03 bis 3 | 0,05 bis 1,5 | 0,1 bis 1 | 0,2 bis 0,6 |
| **Ascorbyl Tetraisopalmitat** | 0,01 bis 8 | 0,03 bis 4 | 0,05 bis 2 | 0,1 bis 1 | 0,2 bis 0,7 |
| **Wasser und weitere Inhaltsstoffe** | add 100 | add 100 | add 100 | add 100 | add 100 |

| | **Formel 26** | **Formel 27** | **Formel 28** | **Formel 29** | **Formel 30** |
|---|---|---|---|---|---|
| **Extrakt Quassia Amara** | 0,001 bis 2 | 0,001 bis 2 | 0,01 bis 0,5 | 0,01 bis 0,5 | 0,05 bis 0,2 |
| **8-Hexadecen-1,16-dicarbonsäure** | 0,01 bis 6 | 0,02 bis 1 | 0,05 bis 0,8 | 0,05 bis 0,5 | 0,1 bis 0,4 |
| **Ascorbinsäure und/oder Ascorbinsäuresalze und/oder Ascorbinsäurederivate** | 0,01 bis 15 | 0,05 bis 5 | 0,1 bis 3 | 0,2 bis 2 | 0,3 bis 1 |
| **Metalloxid** | 0,01 bis 15 | 0,02 bis 10 | 0,05 bis 6 | 0,1 bis 5 | 0,2 bis 3 |
| **Wasser und weitere Inhaltsstoffe** | add 100 | add 100 | add 100 | add 100 | add 100 |

| | **Formel 31** | **Formel 32** | **Formel 33** | **Formel 34** | **Formel 35** |
|---|---|---|---|---|---|
| **Extrakt Quassia Amara** | 0,001 bis 2 | 0,001 bis 2 | 0,01 bis 0,5 | 0,01 bis 0,5 | 0,05 bis 0,2 |
| **8-Hexadecen-1,16-dicarbonsäure** | 0,01 bis 6 | 0,02 bis 1 | 0,05 bis 0,8 | 0,05 bis 0,5 | 0,1 bis 0,4 |
| **Magnesium Ascorbyl Phosphat** | 0,01 bis 7 | 0,03 bis 3 | 0,05 bis 1,5 | 0,1 bis 1 | 0,2 bis 0,6 |
| **Ascorbyl Tetraisopalmitat** | 0,01 bis 8 | 0,03 bis 4 | 0,05 bis 2 | 0,1 bis 1 | 0,2 bis 0,7 |
| **Titandioxid** | 0,01 bis 10 | 0,02 bis 5 | 0,05 bis 4 | 0,1 bis 3 | 0,2 bis 2 |
| **Wasser und weitere Inhaltsstoffe** | add 100 | add 100 | add 100 | add 100 | add 100 |

| | **Formel 36** | **Formel 37** | **Formel 38** | **Formel 39** | **Formel 40** |
|---|---|---|---|---|---|
| **Extrakt Quassia Amara** | 0,001 bis 2 | 0,001 bis 2 | 0,01 bis 0,5 | 0,01 bis 0,5 | 0,05 bis 0,2 |
| **8-Hexadecen-1,16-dicarbonsäure** | 0,01 bis 6 | 0,02 bis 1 | 0,05 bis 0,8 | 0,05 bis 0,5 | 0,1 bis 0,4 |
| **Magnesium Ascorbyl Phosphat** | 0,01 bis 7 | 0,03 bis 3 | 0,05 bis 1,5 | 0,1 bis 1 | 0,2 bis 0,6 |
| **Ascorbyl Tetraisopalmitat** | 0,01 bis 8 | 0,03 bis 4 | 0,05 bis 2 | 0,1 bis 1 | 0,2 bis 0,7 |
| **Metalloxid** | 0,01 bis 15 | 0,02 bis 10 | 0,05 bis 6 | 0,1 bis 5 | 0,2 bis 3 |
| **Emulgatoren** | 0,1 bis 30 | 0,5 bis 25 | 1 bis 20 | 3 bis 15 | 5 bis 10 |
| **Wasser und weitere Inhaltsstoffe** | add 100 | add 100 | add 100 | add 100 | add 100 |

| | **Formel 41** | **Formel 42** | **Formel 43** | **Formel 44** | **Formel 45** |
|---|---|---|---|---|---|
| **Extrakt Quassia Amara** | 0,001 bis 2 | 0,001 bis 2 | 0,01 bis 0,5 | 0,01 bis 0,5 | 0,05 bis 0,2 |
| **8-Hexadecen-1,16-dicarbonsäure** | 0,01 bis 6 | 0,02 bis 1 | 0,05 bis 0,8 | 0,05 bis 0,5 | 0,1 bis 0,4 |
| **Magnesium Ascorbyl Phosphat** | 0,01 bis 7 | 0,03 bis 3 | 0,05 bis 1,5 | 0,1 bis 1 | 0,2 bis 0,6 |
| **Ascorbyl Tetraisopalmitat** | 0,01 bis 8 | 0,03 bis 4 | 0,05 bis 2 | 0,1 bis 1 | 0,2 bis 0,7 |
| **Metalloxid** | 0,01 bis 15 | 0,02 bis 10 | 0,05 bis 6 | 0,1 bis 5 | 0,2 bis 3 |
| **Gemische aus Alkyl-(oligo)-glucosiden und Fettalkoholen** | 0,05 bis 20 | 0,1 bis 15 | 0,5 bis 10 | 1 bis 8 | 2 bis 6 |
| **Cetearyl Isononanoate** | 0,05 bis 20 | 0,1 bis 15 | 0,5 bis 10 | 1 bis 8 | 2 bis 6 |
| **Glyceryl Stearate** | 0,01 bis 6 | 0,05 bis 5 | 0,1 bis 4 | 0,2 bis 3 | 0,5 bis 1,5 |
| **Wasser und weitere Inhaltsstoffe** | add 100 | add 100 | add 100 | add 100 | add 100 |

| | **Formel 46** | **Formel 47** | **Formel 48** | **Formel 49** | **Formel 50** |
|---|---|---|---|---|---|
| **Extrakt Quassia Amara** | 0,001 bis 2 | 0,001 bis 2 | 0,01 bis 0,5 | 0,01 bis 0,5 | 0,05 bis 0,2 |
| **8-Hexadecen-1,16-dicarbonsäure** | 0,01 bis 6 | 0,02 bis 1 | 0,05 bis 0,8 | 0,05 bis 0,5 | 0,1 bis 0,4 |
| **Magnesium Ascorbyl Phosphat** | 0,01 bis 7 | 0,03 bis 3 | 0,05 bis 1,5 | 0,1 bis 1 | 0,2 bis 0,6 |
| **Ascorbyl Tetraisopalmitat** | 0,01 bis 8 | 0,03 bis 4 | 0,05 bis 2 | 0,1 bis 1 | 0,2 bis 0,7 |
| **Metalloxid** | 0,01 bis 15 | 0,02 bis 10 | 0,05 bis 6 | 0,1 bis 5 | 0,2 bis 3 |
| **Gemische aus Alkyl-(oligo)-glucosiden und Fettalkoholen** | 0,05 bis 20 | 0,1 bis 15 | 0,5 bis 10 | 1 bis 8 | 2 bis 6 |
| **Cetearyl Isononanoate** | 0,05 bis 20 | 0,1 bis 15 | 0,5 bis 10 | 1 bis 8 | 2 bis 6 |
| **Glyceryl Stearate** | 0,01 bis 6 | 0,05 bis 5 | 0,1 bis 4 | 0,2 bis 3 | 0,5 bis 1,5 |
| **Schea Butter** | 0,01 bis 15 | 0,05 bis 10 | 0,1 bis 6 | 0,2 bis 4 | 0,5 bis 3 |
| **Wasser und weitere Inhaltsstoffe** | add 100 | add 100 | add 100 | add 100 | add 100 |

| | **Formel 51** | **Formel 52** | **Formel 53** | **Formel 54** | **Formel 55** |
|---|---|---|---|---|---|
| **Extrakt Quassia Amara** | 0,001 bis 2 | 0,001 bis 2 | 0,01 bis 0,5 | 0,01 bis 0,5 | 0,05 bis 0,2 |
| **8-Hexadecen-1,16-dicarbonsäure** | 0,01 bis 6 | 0,02 bis 1 | 0,05 bis 0,8 | 0,05 bis 0,5 | 0,1 bis 0,4 |
| **Magnesium Ascorbyl Phosphat** | 0,01 bis 7 | 0,03 bis 3 | 0,05 bis 1,5 | 0,1 bis 1 | 0,2 bis 0,6 |
| **Ascorbyl Tetraisopalmitat** | 0,01 bis 8 | 0,03 bis 4 | 0,05 bis 2 | 0,1 bis 1 | 0,2 bis 0,7 |
| **Metalloxid** | 0,01 bis 15 | 0,02 bis 10 | 0,05 bis 6 | 0,1 bis 5 | 0,2 bis 3 |
| **Gemische aus Alkyl-(oligo)-glucosiden und Fettalkoholen** | 0,05 bis 20 | 0,1 bis 15 | 0,5 bis 10 | 1 bis 8 | 2 bis 6 |
| **Cetearyl Isononanoate** | 0,05 bis 20 | 0,1 bis 15 | 0,5 bis 10 | 1 bis 8 | 2 bis 6 |
| **Glyceryl Stearate** | 0,01 bis 6 | 0,05 bis 5 | 0,1 bis 4 | 0,2 bis 3 | 0,5 bis 1,5 |
| **Schea Butter** | 0,01 bis 15 | 0,05 bis 10 | 0,1 bis 6 | 0,2 bis 4 | 0,5 bis 3 |
| **Ethylhexyl triazone** | 0,01 bis 8 | 0,05 bis 5 | 0,1 bis 5 | 0,2 bis 3 | 0,5 bis 1,5 |
| **Wasser und weitere Inhaltsstoffe** | add 100 | add 100 | add 100 | add 100 | add 100 |

| | **Formel 56** | **Formel 57** | **Formel 58** | **Formel 59** | **Formel 60** |
|---|---|---|---|---|---|
| **Extrakt Quassia Amara** | 0,001 bis 2 | 0,001 bis 2 | 0,01 bis 0,5 | 0,01 bis 0,5 | 0,05 bis 0,2 |
| **8-Hexadecen-1,16-dicarbonsäure** | 0,01 bis 6 | 0,02 bis 1 | 0,05 bis 0,8 | 0,05 bis 0,5 | 0,1 bis 0,4 |
| **Magnesium Ascorbyl Phosphat** | 0,01 bis 7 | 0,03 bis 3 | 0,05 bis 1,5 | 0,1 bis 1 | 0,2 bis 0,6 |
| **Ascorbyl Tetraisopalmitat** | 0,01 bis 8 | 0,03 bis 4 | 0,05 bis 2 | 0,1 bis 1 | 0,2 bis 0,7 |
| **Metalloxid** | 0,01 bis 15 | 0,02 bis 10 | 0,05 bis 6 | 0,1 bis 5 | 0,2 bis 3 |
| **Montanov 202** | 0,05 bis 16 | 0,1 bis 15 | 0,5 bis 10 | 1 bis 8 | 2 bis 6 |
| **Cetiol^{®} SN** | 0,05 bis 16 | 0,1 bis 15 | 0,5 bis 10 | 1 bis 8 | 2 bis 6 |
| **Cutina MD** | 0,01 bis 6 | 0,05 bis 5 | 0,1 bis 4 | 0,2 bis 3 | 0,5 bis 1,5 |
| **Cetiol^{®} SB 45** | 0,01 bis 12 | 0,05 bis 10 | 0,1 bis 6 | 0,2 bis 4 | 0,5 bis 3 |
| **Uvinul T 150** | 0,01 bis 8 | 0,05 bis 5 | 0,1 bis 5 | 0,2 bis 3 | 0,5 bis 1,5 |
| **Pigment** | 0,01 bis 10 | 0,05 bis 8 | 0,2 bis 6 | 0,5 bis 5 | 1 bis 4 |
| **Wasser und weitere Inhaltsstoffe** | add 100 | add 100 | add 100 | add 100 | add 100 |

| | **Formel 61** | **Formel 62** | **Formel 63** | **Formel 64** | **Formel 65** |
|---|---|---|---|---|---|
| **Extrakt Quassia Amara** | 0,001 bis 2 | 0,001 bis 2 | 0,01 bis 0,5 | 0,01 bis 0,5 | 0,05 bis 0,2 |
| **8-Hexadecen-1,16-dicarbonsäure** | 0,01 bis 6 | 0,02 bis 1 | 0,05 bis 0,8 | 0,05 bis 0,5 | 0,1 bis 0,4 |
| **Magnesium Ascorbyl Phosphat** | 0,01 bis 7 | 0,03 bis 3 | 0,05 bis 1,5 | 0,1 bis 1 | 0,2 bis 0,6 |
| **Ascorbyl Tetraisopalmitat** | 0,01 bis 8 | 0,03 bis 4 | 0,05 bis 2 | 0,1 bis 1 | 0,2 bis 0,7 |
| **Metalloxid** | 0,01 bis 10 | 0,02 bis 10 | 0,05 bis 6 | 0,1 bis 5 | 0,2 bis 3 |
| **Montanov 202** | 0,05 bis 13 | 0,1 bis 12 | 0,5 bis 10 | 1 bis 8 | 2 bis 6 |
| **Cetiol^{®} SN** | 0,05 bis 13 | 0,1 bis 12 | 0,5 bis 10 | 1 bis 8 | 2 bis 6 |
| **Cutina MD** | 0,01 bis 6 | 0,05 bis 5 | 0,1 bis 4 | 0,2 bis 3 | 0,5 bis 1,5 |
| **Cetiol^{®} SB 45** | 0,01 bis 12 | 0,05 bis 10 | 0,1 bis 6 | 0,2 bis 4 | 0,5 bis 3 |
| **Uvinul T 150** | 0,01 bis 6 | 0,05 bis 5 | 0,1 bis 5 | 0,2 bis 3 | 0,5 bis 1,5 |
| **Pigment** | 0,01 bis 8 | 0,05 bis 8 | 0,2 bis 6 | 0,5 bis 5 | 1 bis 4 |
| **Parfum** | 0,01 bis 5 | 0,05 bis 5 | 0,1 bis 4 | 0,1 bis 3 | 0,2 bis 2 |
| **Konservierungsstoff** | 0,01 bis 8 | 0,05 bis 8 | 0,1 bis 6 | 0,2 bis 5 | 0,5 bis 3 |
| **Verdickungsmittel** | 0,01 bis 5 | 0,05 bis 5 | 0,1 bis 4 | 0,1 bis 3 | 0,2 bis 2 |
| **Wasser und weitere Inhaltsstoffe** | add 100 | add 100 | add 100 | add 100 | add 100 |

| | **Formel 66** |
|---|---|
| **Quassia Amara A 00264** | 0,05 bis 0,3 |
| **ODA White** | 0,1 bis 0,4 |
| **Nikkol VC IP** | 0,1 bis 1 |
| **Nikkol VC PMG C** | 0,1 bis 1 |
| **RonaFlair^{®} Extender W** | 0,1 bis 2 |
| **RonaFlair^{®} Balance Gold** | 0,1 bis 2 |
| **Montanov 202** | 2 bis 6 |
| **Cetiol^{®} SN** | 2 bis 6 |
| Cutina **MD** | 0,5 bis 1,5 |
| **Cetiol^{®} SB 45** | 0,5 bis 3 |
| **Uvinul T 150** | 0,5 bis 1,5 |
| **Pigment** | 1 bis 4 |
| **Parfum** | 0,2 bis 2 |
| **Konservierungsstoff** | 0,5 bis 3 |
| **Verdickungsmittel** | 0,2 bis 2 |
| **Wasser und weitere Inhaltsstoffe** | add 100 |

Die erfindungsgemäßen Mittel nach Formel 66 wurden in einem Gebrauchstest unter dermatologischer Kontrolle an 50 Probandinnen mit Altersflecken im Gesicht geprüft. Die Testpersonen sollten dabei die Mittel über insgesamt 4 Wochen mindestens zweimal täglich zur Gesichtspflege anwenden. Der dermatologische Gebrauchstest der erfindungsgemäßen Mittel im Vergleich zur Mittelvariante, die kein Quassia amara Extrakt enthält, ergibt, dass die erfindungsgemäßen Mittel mit Extrakt aus Quassia amara viel bessere Aufhellungseffekte gegen Hautflecken zeigen und die Haut wirksamer gegen das Auftreten neuer Hautflecken schützten.

### Verwendete Rohstoffe

| **Handelsname** | **INCI**-**Bezeichnung** | **Lieferant/Hersteller** |
|---|---|---|
| **Quassia Amara A 00264** | Quassia Amara Wood Extract | BASF |
| **Cetiol^{®} SB 45** | Butyrospermum Parkii (Schea Butter) | Cognis |
| **Cetiol^{®} SN** | Cetearyl Isononanoate | Cognis |
| **Cutina MD** | Glyceryl Stearate | Cognis |
| **RonaFlair^{®} Extender W** | MICA, Cl 77891 (Titanium Dioxide) | Merck |
| **RonaFlair^{®} Balance Gold** | Mica, Cl 77891 (Titanium Dioxide), Tin Oxide | Merck |
| **Montanov 202** | Arachidyl Alcohol, Behenyl alcohol, Arachidyl Glucoside | Seppic |
| **Nikkol VC IP** | Ascorbyl Tetraisopalmitate | Nikko Chemicals |
| **Nikkol VC PMG C** | Magnesium Ascorbyl Phosphate | Nikko Chemicals |
| **ODA White** | Octadecenedioic Acid, BHT | Croda |
| **Uvinul T 150** | Ethylhexyl triazone | BASF |

## Patentansprüche

1. Kosmetisches oder dermatologisches Mittel, enthaltend eine Kombination aus mindestens einem der unter a) und mindestens einem der unter b) aufgeführten Wirkstoffe:
a) Extrakt aus Quassia Amara, welcher durch Extraktion von Quassia amara Holz mit Wasser erhältlich ist;
b) gesättigte oder ungesättigte, lineare (C9- bis C18)-Dicarbonsäure, insbesondere 8-Hexadecen-1,16-dicarbonsäure oder 7-Tetradecen-1,14-dicarbonsäure oder 9-Octadecen-1,18-dicarbonsäure oder 6-Dodecen-1,12-dicarbonsäure oder 5-Decen-1,10-dicarbonsäure oder Decandisäure (Sebacinsäure) oder Nonandisäure (Azelainsäure), ganz besonders bevorzugt 8-Hexadecen-1,16-dicarbonsäure.

2. Mittel nach Anspruch 1, **dadurch gekennzeichnet, dass** es, bezogen auf sein Gesamtgewicht, insgesamt 0,001 bis 2,0 Gew.-%, vorzugsweise 0,01 bis 0,5 Gew.-% und insbesondere 0,05 bis 0,2 Gew.-% mindestens eines Extraktes aus Quassia Amara enthält.

3. Mittel nach einem der Ansprüche 1 - 2, **dadurch gekennzeichnet, dass** es, bezogen auf sein Gesamtgewicht, die gesättigte oder ungesättigte, lineare (C9- bis C18)-Dicarbonsäure, insbesondere die 8-Hexadecen-1,16-dicarbonsäure, in einer Gesamtmenge von 0,01 - 10 Gew.-%, vorzugsweise 0,05 bis 1 Gew.-% und insbesondere 0,1 bis 0,5 Gew.-%, enthält.

4. Mittel nach einem der Ansprüche 1 - 3, **dadurch gekennzeichnet, dass** das Gewichtsverhältnis des Gesamtgehalts an Extrakt aus Quassia Amara zur Gesamtmenge der gesättigten oder ungesättigten, linearen (C9- bis C18)-Dicarbonsäure 1 : 10 bis 5: 1, vorzugsweise 1 : 5 bis 2 : 1 und insbesondere 1 : 3 bis 1 : 1, beträgt.

5. Mittel nach einem der Ansprüche 1 - 4, **dadurch gekennzeichnet, dass** es zusätzlich insgesamt 0,01 bis 5 Gew.-%, vorzugsweise 0,1 bis 1 Gew.-%, an mindestens einer weiteren hautaufhellenden Substanz enthält, die ausgewählt ist aus der Gruppe, bestehend aus Ascorbinsäure, Ascorbinsäuresalzen, Ascorbinsäureglycosiden, Ascorbinsäureglycosidsalzen, Ascorbinsäureestern von organischen Säuren, den Salzen der Ascorbinsäureester von organischen Säuren, Ascorbinsäureestern von anorganischen Säuren, den Salzen der Ascorbinsäureester von anorganischen Säuren, sowie Mischungen der vorgenannten Substanzen, insbesondere Ascorbinsäure.

6. Mittel nach einem der Ansprüche 1 - 5, **dadurch gekennzeichnet, dass** es mindestens ein Metalloxid, vorzugsweise ein Metalloxid als hautaufhellenden Wirkstoff, enthält.

7. Mittel nach Anspruch 6, **dadurch gekennzeichnet, dass** es, bezogen auf sein Gesamtgewicht, insgesamt 0,01 bis 5 Gew.-%, vorzugsweise 0,1 bis 3 Gew.-% Metalloxid, bevorzugt Metalloxid als hautaufhellenden Wirkstoff enthält.

8. Mittel nach einem der Ansprüche 1 - 7, **dadurch gekennzeichnet, dass** es 10 bis 80 Gew.-%, vorzugsweise 20 bis 75 Gew.-%, weiter bevorzugt 30 bis 70 Gew.-%, noch weiter bevorzugt 35 bis 60 Gew.-% und insbesondere 40 bis 60 Gew.-% Wasser enthält.

9. Nicht-therapeutische, kosmetische Verwendung eines kosmetischen oder dermatologischen Mittels nach einem der Ansprüche 1 - 8 zur Aufhellung der Hautfarbe, zur Reduzierung der Pigmentierung der Haut, zum Ausgleich des Erscheinungsbildes einer ungleichmäßigen Hautpigmentierung und/oder zur Aufhellung von Altersflecken oder Sommersprossen der Haut.

10. Nicht-therapeutisches, kosmetisches Verfahren zur Aufhellung der Hautfarbe, zur Reduzierung der Pigmentierung der Haut, zum Ausgleich des Erscheinungsbildes einer ungleichmäßigen Hautpigmentierung und/oder zur Aufhellung von Altersflecken oder Sommersprossen der Haut, **dadurch gekennzeichnet, dass** ein kosmetisches Mittel, enthaltend eine Kombination aus mindestens einem der unter a) und mindestens einem der unter b) aufgeführten Wirkstoffe:
a) Extrakt aus Quassia Amara, welcher durch Extraktion von Quassia amara Holz mit Wasser erhältlich ist;
b) gesättigte oder ungesättigte, lineare (C9- bis C18)-Dicarbonsäure, insbesondere 8-Hexadecen-1,16-dicarbonsäure oder 7-Tetradecen-1,14-dicarbonsäure oder 9-Octadecen-1,18-dicarbonsäure oder 6-Dodecen-1,12-dicarbonsäure oder 5-Decen-1,10-dicarbonsäure oder Decandisäure (Sebacinsäure) oder Nonandisäure (Azelainsäure), ganz besonders bevorzugt 8-Hexadecen-1,16-dicarbonsäure,
auf die Haut aufgetragen wird.

## Claims

1. A cosmetic or dermatological agent containing a combination of at least one of the active ingredients listed under a) and at least one of the active ingredients listed under b):
a) Quassia amara extract, which can be obtained by extraction of Quassia amara wood using water;
b) saturated or unsaturated, linear (C9- to C18)-dicarboxylic acid, in particular 8-hexadecene-1,16-dicarboxylic acid or 7-tetradecene-1,14-dicarboxylic acid or 9-octadecene-1,18-dicarboxylic acid or 6-dodecene-1,12-dicarboxylic acid or 5-decene-1,10-dicarboxylic acid or decanedioic acid (sebacic acid) or nonanedioic acid (azelaic acid), particularly preferably 8-hexadecene-1,16-dicarboxylic acid.

2. The agent according to claim 1, **characterized in that** it contains, based on its total weight, a total of from 0.001 to 2.0 wt.%, preferably from 0.01 to 0.5 wt.% and in particular from 0.05 to 0.2 wt.%, of at least one Quassia amara extract.

3. The agent according to either claim 1 or claim 2, **characterized in that** it contains, based on its total weight, saturated or unsaturated, linear (C9- to C18)-dicarboxylic acid, in particular 8-hexadecene-1,16-dicarboxylic acid, in a total amount of from 0.01 to 10 wt.%, preferably from 0.05 to 1 wt.% and in particular from 0.1 to 0.5 wt.%.

4. The agent according to one of claims 1 to 3, **characterized in that** the weight ratio of the total content of Quassia amara extract to the total amount of saturated or unsaturated, linear (C9-to C18)-dicarboxylic acid is from 1:10 to 5:1, preferably from 1:5 to 2:1 and in particular from 1:3 to 1:1.

5. The agent according to one of claims 1 to 4, **characterized in that** it additionally contains a total of from 0.01 to 5 wt.%, preferably from 0.1 to 1 wt.%, of at least one further skin-lightening substance selected from the group consisting of ascorbic acid, ascorbic acid salts, ascorbic acid glycosides, ascorbic acid glycoside salts, ascorbic acid esters of organic acids, ascorbic acid ester salts of organic acids, ascorbic acid esters of inorganic acids, ascorbic acid ester salts of inorganic acids, and mixtures of the aforementioned substances, in particular ascorbic acid.

6. The agent according to one of claims 1 to 5, **characterized in that** it contains at least one metal oxide, preferably a metal oxide as the skin-lightening active ingredient.

7. The agent according to claim 6, **characterized in that** it contains, based on its total weight, a total of from 0.01 to 5 wt.%, preferably from 0.1 to 3 wt.% metal oxide, preferably metal oxide as the skin-lightening active ingredient.

8. The agent according to one of claims 1 to 7, **characterized in that** it contains from 10 to 80 wt.%, preferably from 20 to 75 wit.%, more preferably from 30 to 70 wt.%, even more preferably from 35 to 60 wt.% and in particular from 40 to 60 wt.%, water.

9. The non-therapeutic, cosmetic use of a cosmetic or dermatological agent according to one of claims 1 to 8 for lightening skin colour, reducing pigmentation of the skin, balancing the appearance of non-uniform skin pigmentation and/or lightening age marks or freckles on the skin.

10. The non-therapeutic, cosmetic method of lightening skin colour, reducing pigmentation of the skin, balancing the appearance of non-uniform skin pigmentation and/or lightening age marks and freckles on the skin, **characterized in that** a cosmetic agent containing a combination of at least one of the active ingredients listed under a) and one of the active ingredients listed under b):
a) Quassia amara extract, which can be obtained by extraction of Quassia amara wood using water;
b) saturated or unsaturated, linear (C9- to C18)-dicarboxylic acid, in particular 8-hexadecene-1,16-dicarboxylic acid or 7-tetradecene-1,14-dicarboxylic acid or 9-octadecene-1,18-dicarboxylic acid or 6-dodecene-1,12-dicarboxylic acid or 5-decene-1,10-dicarboxylic acid or decanedioic acid (sebacic acid) or nonanedioic acid (azelaic acid), particularly preferably 8-hexadecene-1,16-dicarboxylic acid,
is applied to the skin.

## Revendications

1. Produit cosmétique ou dermatologique contenant une combinaison d'au moins une parmi les substances actives produites a) et d'au moins une parmi les substances actives produites b) :
a) extrait de quassia amara disponible par extraction du bois de quassia amara par l'eau ;
b) acide dicarboxylique saturé ou insaturé linéaire (C9 à C18), en particulier acide 8-hexadécèn-1,16-dicarboxylique, acide 7-tétradécèn-1,14-dicarboxylique, acide 9-octadécèn-1,18-dicarboxylique, acide 6-dodécèn-1,12-dicarboxylique, acide 5-décèn-1,10-dicarboxylique, acide décanedicarboxylique (acide sébacinique) ou acide nonanedicarboxylique (acide azélaïnique), tout particulièrement l'acide 8-hexadécèn-1,16-dicarboxylique.

2. Produit selon la revendication 1, **caractérisé en ce qu'**il contient, rapporté à son poids total, en tout 0,001 % à 2,0 % en poids, de préférence 0,01 % à 0,5 % en poids et en particulier 0,05 % à 0,2% en poids d'au moins un extrait de quassia amara.

3. Produit selon la revendication 1 ou 2, **caractérisé en ce qu'**il contient, rapporté à son poids total, l'acide dicarboxylique saturé ou insaturé linéaire (C9 à C18), en particulier acide 8-hexadécèn-1,16-dicarboxylique, à hauteur de 0,01 à 10 % en poids, de préférence 0,05 % à 1 % en poids et en particulier 0,1 % à 0,5% en poids.

4. Produit selon la revendication 1 à 3, **caractérisé en ce que** le rapport pondéral de la teneur totale en extrait de quassia amara par rapport à la quantité totale de l'acide dicarboxylique saturé ou insaturé linéaire (C9 à C18) se monte à entre 1:10 et 5:1, de préférence entre 1:5 et 2:1 et en particulier entre 1:3 et 1:1.

5. Produit selon la revendication 1 à 4, **caractérisé en ce qu'**il contient en plus au total 0,01 % à 5 % en poids, de préférence 0,1 % à 1 % en poids, d'au moins une autre substance éclaircissant la peau, choisie dans le groupe constitué de l'acide ascorbique, les sels de l'acide ascorbique, les glycosides de l'acide ascorbique, les sels de glycosides de l'acide ascorbique, les esters d'acide ascorbique d'acides organiques, les sels d'esters d'acide ascorbique d'acides organiques, les esters d'acide ascorbique d'acides minéraux, les sels d'esters d'acide ascorbique d'acides minéraux, ainsi que des mélanges des substances susmentionnées, en particulier l'acide ascorbique.

6. Produit selon la revendication 1 à 5, **caractérisé en ce qu'**il contient au moins un oxyde métallique, de préférence un oxyde métallique substance éclaircissante pour la peau.

7. Produit selon la revendication 6, **caractérisé en ce qu'**il contient, rapporté à son poids total, en tout 0,01 % à 5 % en poids, de préférence 0,1% à 3% en poids d'un oxyde métallique, de préférence un oxyde métallique substance éclaircissante pour la peau.

8. Produit selon la revendication 1 à 7, **caractérisé en ce qu'**il contient 10 % à 80 % en poids, de préférence 20 % à 75 % en poids, plus préférentiellement 30 % à 70 % en poids, encore plus préférentiellement 35 % à 60 % en poids et en particulier 40 % à 60 % en poids d'eau.

9. Utilisation cosmétique non-thérapeutique d'un produit cosmétique ou dermatologique selon une des revendications 1 à 8 pour éclaircir la couleur de la peau, pour réduire la pigmentation de la peau, pour égaliser l'aspect d'une pigmentation irrégulière de la peau et/ou pour éclaircir des taches de vieillissement et des taches de rousseur de la peau.

10. Procédé cosmétique non-thérapeutique d'éclaircissement de la couleur de la peau, de réduction de la pigmentation de la peau, d'égalisation de l'aspect d'une pigmentation irrégulière de la peau et/ou d'éclaircissement des taches de vieillissement et des taches de rousseur de la peau, **caractérisé en ce qu'**un produit cosmétique contenant une combinaison d'au moins une parmi les substances actives produites a) et d'au moins une parmi les substances actives produites b) :
a) extrait de quassia amara disponible par extraction du bois de quassia amara par l'eau ;
b) acide dicarboxylique saturé ou insaturé linéaire (C9 à C18), en particulier acide 8-hexadécèn-1,16-dicarboxylique, acide 7-tétradécèn-1,14-dicarboxylique, acide 9-octadécèn-1,18-dicarboxylique, acide 6-dodécèn-1,12-dicarboxylique, acide 5-décèn-1,10-dicarboxylique, acide décanedicarboxylique (acide sébacinique) ou acide nonanedicarboxylique (acide azélaïnique), tout particulièrement l'acide 8-hexadécèn-1,16-dicarboxylique,
est appliqué sur la peau.
